(19) Europäisches Patentamt
European Patent Office
Office européen des brevets

(11) **EP 0 701 809 A1**

(12) **DEMANDE DE BREVET EUROPEEN**

(43) Date de publication:
**20.03.1996 Bulletin 1996/12**

(51) Int Cl.⁶: **A61K 7/00**

(21) Numéro de dépôt: **95402034.3**

(22) Date de dépôt: **08.09.1995**

(84) Etats contractants désignés:
**AT BE CH DE ES FR GB IT LI NL SE**

(30) Priorité: **13.09.1994 FR 9410924**

(71) Demandeur: **L'OREAL**
**F-75008 Paris (FR)**

(72) Inventeurs:
• **Marchi-Lemann, Patricia**
  **F-75008 Paris (FR)**
• **Colin, Christian**
  **F-75020 Paris (FR)**

(74) Mandataire: **Casalonga, Axel**
  **BUREAU D.A. CASALONGA - JOSSE**
  **Morassistrasse 8**
  **D-80469 München (DE)**

(54) **Composition cosmétique prévenant ou atténuant la photo-réactivité de nanopigments de dioxyde de titane**

(57) Composition cosmétique, caractérisée par le fait qu'elle contient des nanopigments de dioxyde de titane enrobés au moins avec des dérivés d'aluminium dans un milieu cosmétiquement acceptable, peu polaire, ayant une conductivité inférieure à 100 μSiemens et comportant au moins une phase grasse.

## Description

La présente invention a pour objet des compositions cosmétiques contenant des nanopigments d'oxyde de titane, ces compositions présentant l'avantage d'être particulièrement stables à la lumière solaire.

Le dioxyde de titane sous forme de nanopigments est bien connu pour sa propriété d'absorption de la lumière, en particulier dans le domaine des UV. C'est pour cela qu'il est utilisé dans des compositions cosmétiques comme filtre minéral, en remplacement ou en association avec des filtres organiques.

L'absorption dans l'UV engendre cependant des phénomènes indésirables d'oxydo-réduction de l'oxyde de titane, qui se manifestent par un changement de couleur de la composition. On note en effet un bleuissement ou un jaunissement et une oxydation des autres composants.

Sans que cette explication soit limitative, on pense que la lumière catalyse la réduction du titane IV (blanc) en titane III (bleu). Ces réactions pouvant se produire même à très faible pourcentage d'oxyde de titane.

On recherche, de ce fait, des moyens de protéger de telles compositions pour éviter notamment ces problèmes d'oxydation et de changement de couleur, indésirables dans le domaine cosmétique.

Toutefois, le dioxyde de titane, utilisé comme agent de protection contre le rayonnement ultraviolet, doit continuer à jouer son rôle protecteur.

On a déjà décrit, dans l'état de la technique, l'utilisation des nanopigments d'oxyde de titane dans des compositions solaires en association avec des filtres solaires, en particulier US-A-5.028.417 ou de façon isolée JP-A-0038436.

Le problème de la photoréactivité de l'oxyde de titane a également été soulevé, et le brevet GB-A-2.217.987 propose l'utilisation de nanopigments recouverts par un dérivé d'aluminium en vue de minimiser la réduction photo-induite par le dioxyde de titane.

Le brevet JP-03115211 propose un traitement de surface par des sels de silice et d'alumine qui permettrait une meilleure dispersibilité des nanopigments ainsi qu'une atténuation de la dénaturation des huiles environnantes.

Enfin, le PCT WO 9009777 envisage l'ajout de sels de phosphate, soit en traitement de surface des pigments, soit par simple introduction dans la formule pour réduire la photocatalyse.

L'objectif poursuivi par ces différentes solutions préconisées dans l'état de la technique, consiste essentiellement à traiter le dioxyde de titane de façon à le rendre inerte vis-à-vis de la lumière. On a cependant constaté que de tels traitements ne sont pas toujours suffisants. C'est ainsi que l'on a également envisagé d'utiliser des antioxydants en présence de nanopigments de dioxyde de titane.

Les inventeurs ont découvert qu'en utilisant des nanopigments de $TiO_2$ protégés par un traitement de surface constitué de sels d'aluminium et de silicium dans un milieu peu polaire, il était possible d'obtenir des compositions à base de dioxyde de titane particulièrement stables aux rayonnements UV et ne provoquant pas l'oxydation des autres composants présents dans la composition.

L'invention a donc pour objet une nouvelle composition cosmétique à base de nanopigments de dioxyde de titane.

Un autre objet de l'invention est constitué par le procédé du traitement cosmétique mettant en oeuvre de telles compositions.

L'invention a également pour objet un procédé de prévention ou d'atténuation de la photo-réactivité de nanopigments de dioxyde de titane.

D'autres objets de l'invention apparaîtront à la lecture de la description et des exemples qui suivent.

La composition cosmétique conforme à l'invention est essentiellement caractérisée par le fait qu'elle contient des nanopigments de dioxyde de titane protégés par un traitement de surface avec au moins un dérivé d'aluminium dans un milieu cosmétiquement acceptable, peu polaire, ayant une conductivité inférieure à 100 µSiemens et comportant au moins une phase grasse.

On utilise de préférence un traitement de surface avec des dérivés d'aluminium et de silicium.

Les dérivés d'aluminium et de silicium sont plus particulièrement choisis parmi les oxydes d'aluminium et de silicium. Leur proportion est telle que le pourcentage total d'aluminium et de silicium présent dans le revêtement représente au moins 5% en poids par rapport au poids de dioxyde de titane. La quantité d'aluminium est de préférence supérieure à la quantité de silicium. Le taux d'enrobage est de préférence compris entre 5 et 30% en poids par rapport au poids de dioxyde de titane et en particulier entre 5 et 20%.

On utilise de préférence un mélange d'oxyde d'aluminium et d'oxyde de silicium. Le rapport $Al_2O_3/SiO_2$ est de préférence supérieur ou égal à 3 et inférieur ou égal à 8. $Al_2O_3$ étant présent dans une quantité supérieure par rapport à la quantité de $SiO_2$ qui est utilisée dans une quantité supérieure ou égale à 1% en poids par rapport a poids de dioxyde de titane.

Les nanopigments de dioxyde de titane protégé par un traitement de surface avec au moins un dérivé d'aluminium et de préférence avec des dérivés d'aluminium et de silicium, peuvent faire l'objet dans une forme de réalisation de l'invention, d'un enrobage de surface supplémentaire tel que par des silicones, des acides aminés, des sels d'acides gras autres que des sels d'aluminium, de la lécithine, ...

Un tel enrobage supplémentaire permet notamment de faciliter la dispersion du dioxyde de titane dans le milieu.

Le milieu cosmétiquement acceptable est peu polaire, il a une conductivité inférieure à 100 µSiemens mesurée à l'aide d'un multiparamètre M90 CHECKMATE de la Société METTLER-TOLEDO.

Le milieu cosmétiquement acceptable comportant au moins une phase brasse, utilisé conformément à l'invention, peut être anhydre ou hydraté et se présente dans ce dernier cas sous forme d'émulsion eau-dans-huile (E/H) ou huile-dans-eau (H/E).

Cette phase grasse est constituée par une huile ou un mélange d'huiles ou de corps gras.

Les huiles ou mélanges d'huiles ou de corps gras doivent être peu ou pas polaire. De préférence, la constante diélectrique de l'huile ou des mélanges mis en oeuvre doit être inférieure à 3 et de préférence inférieure à 2,5.

L'indice de polarité est de préférence supérieur à 24 mN/m et en particulier à 35 mN/m.

Dans le cas des mélanges d'huiles, il est possible d'utiliser dans le mélange une huile plus polaire, à la condition que le mélange final soit peu polaire, comme défini ci-dessus.

On utilise généralement des huiles de type carboné et peu polaire. On peut citer les hydrocarbures comme l'huile de vaseline, le squalane, les esters et triglycérides courts, c'est-à-dire comportant 1 à 6 atomes de carbone dans le radical alkyle, tel que l'adipate d'isopropyle, le myristate d'isopropyle, le palmitate d'isopropyle ou encore un benzoate d'alcools en $C_{12}C_{15}$. On peut également utiliser des huiles telles que l'octyldodécanol ou l'alcool myristique oxypropyléné avec 1 à 5 moles d'oxyde de propylène.

Les compositions conformes à l'invention contiennent de préférence entre 0,5 et 30% et de préférence de 0,5 à 10% en poids de dioxyde de titane, ayant une granulométrie inférieure ou égale à 200 nm et de préférence inférieure à 100 nm, enrobé de dérivés d'aluminium et de silicium. La phase grasse, peu polaire, cosmétiquement acceptable, est généralement présente dans des proportions de 5 à 99,5% en poids par rapport au poids total de la composition. Ces proportions sont de préférence comprises entre 5 et 50% pour les émulsions.

Les compositions conformes à l'invention peuvent également contenir des sels tels que NaCl, $MgSO_4$. Ces sels doivent cependant être utilisés dans des proportions telles qu'elles n'augmentent pas la conductivité du milieu, à une valeur supérieure à 100 µSiemens.

Ces compositions peuvent également contenir des agents antioxydants tels que l'extrait d'huile de sésame, la superoxyde dismutase, la caféine, le cytochrome C, le tertiobutyl hydroxytoluène, l'acide diéthylène triamine pentaacétique, l'éthoxyquine, la lactoperoxydase, l'acide déhydroxy ascorbique. L'éthoxyquine étant particulièrement préférée.

La demanderesse a constaté que les compositions étaient particulièrement stables lorsqu'elles contiennent en plus des nanopigments de dioxyde de titane comportant un revêtement de surface constitué par des dérivés d'aluminium et de silicium dans un milieu peu polaire, de l'éthoxyquine comme anti-oxydant.

Les proportions en agents anti-oxydants sont généralement comprises entre 0,01 et 2% et de préférence entre 0,01 et 0,05%.

Ces compositions sont généralement préparées en introduisant les nanopigments dans la phase grasse de la composition, ce qui permet une meilleure dispersion des pigments compte-tenu de plus grandes forces de cisaillement mises en jeu dans les milieux gras plus visqueux.

Ces compositions peuvent également contenir d'autres adjuvants habituellement utilisés en cosmétique, sous réserve qu'ils n'augmentent pas la conductivité de la composition à une valeur supérieure à 100 µSiemens. Elles peuvent se présenter sous forme d'émulsion, de crème, d'huile éventuellement épaissie. Ces compositions peuvent être conditionnées en aérosol et se présenter sous forme de mousse.

L'invention a également pour objet un procédé de prévention ou d'atténuation de la photo-réactivité de nanopigments de dioxyde de titane dans les compositions cosmétiques. Ce procédé est essentiellement caractérisé par le fait que l'on enrobe les nanopigments de dioxyde de titane avec un dérivé d'aluminium et de silicium tel que défini ci-dessus et qu'on introduit ces nanopigments enrobés dans un milieu cosmétiquement acceptable, peu polaire, ayant une conductivité inférieure à 100 µSiemens. Ce milieu comprend de préférence une phase grasse et présente les caractéristiques telles que définies ci-dessus.

Un autre objet de l'invention est constitué par le procédé de traitement cosmétique consistant à appliquer une composition telle que définie ci-dessus, sur la peau. Cette composition peut être utilisée pour la protection solaire ainsi que comme produit de maquillage, tels que fond de teint, mascara, etc...

Les exemples qui suivent sont destinés à illustrer l'invention sans pour autant présenter un caractère limitatif.

EXEMPLE 1

On prépare la composition suivante :

| | | | | |
|---|---|---|---|---|
| **Phase grasse** | Alcool cétylstéarylique et alcool cétylstéarylique oxyéthylène | 7 | % |
| | Glycéryl distéarate et glycéryl stéarate | 2 | % |
| | Alcool cétylique | 1,5 | % |
| | Polydiméthylsiloxane | 1,5 | % |
| | P-hydroxybenzoate de butyle | 0,2 | % |
| | Huile de vaseline | 15 | % |
| **Phase aqueuse** | Glycérine | 20 | % |
| | Eau | 45,6 | % |
| **Conservateurs** | Imidazolidinyl urée | 0,2 | % |
| | Eau | 2 | % |
| **Pigment** | MT 100T * | 5 | % |

\* **MT 100T** est un nanodioxyde de titane (Rutile traité avec du stéarate d'aluminium (80/20) ayant une granulométrie moyenne de 15 nm, vendu par la Société TAYCA.

La conductivité du milieu est de 30 µSiemens.

**Mode opératoire :**

Les pigments sont incorporés dans la phase grasse (chauffée à 80°C environ) sous agitation au MORITZ.
Cette phase est ensuite introduite dans la phase aqueuse (80°C) sous agitation au MORITZ.
On poursuit cette agitation pendant 5 minutes, puis lorsque l'émulsion a atteint environ 40°C, on ajoute les conservateurs.
L'agitation est arrêtée lorsque l'émulsion est à température ambiante. On peut finir cette agitation à la pâte.

EXEMPLE 2

On prépare la composition suivante :

|  |  |  |  |
|---|---|---|---|
| **Phase grasse** | Alcool cétylstéarylique et alcool cétylstéarylique oxyéthylène | 5 | % |
|  | Glycéryl distéarate et glycéryl stéarate | 1 | % |
|  | Alcool cétylique | 1 | % |
|  | Huile (différentes huiles ont été utilisées, conforme Tableau I) | 6 | % |
|  | Vitamine F * | 6 | % |
| **Pigment** | MT 100T | 5 | % |
| **Phase aqueuse** | Eau | qsp | 100 |
|  |  | 45,6 | % |

\* **Vitamine F** : Acide 9-12 linoléique cis-cis (50%) avec présence d'acides oléiques (5%) et 9-11 octadécadiénoïque (40%) stabilisé.

Cette composition est préparée comme décrit à l'exemple 1.

Le **TABLEAU I** qui suit démontre l'importance du milieu peu polaire, l'huile de jojoba polaire a un effet négatif sur la stabilité de la vitamine F, en présence du pigment.

ΔE exprime la différence de coloration entre l'échantillon au temps O (avant exposition) et après exposition.

Il est déterminé par la formule $\Delta E = \sqrt{\Delta L^2 + \Delta a^2 + \Delta b^2}$, dans laquelle le paramètre $\underline{L}$ définit le caractère plus ou moins sombre, $\underline{a}$: la nuance bleu ou vert, $\underline{b}$: la nuance jaune ou rouge, ou système Lab.

## TABLEAU I

### EVOLUTION DE LA VITAMINE F EN PRESENCE DE DIVERSES HUILES

| Compositions testées | Pourcentage d'activation (+) ou d'inhibition (-) de l'oxydation de la vitamine F après différents traitements | | ΔE BLEUISSEMENT | |
|---|---|---|---|---|
| | 1 h UVA +5 j 37° | 24 h UVA | Après 1 h UVA | Après 24 h UVA |
| 1 Huile de vaseline + MT100T | - 67 % | - 63 % | 2,99 | 13,96 |
| 2 Finsolv LIN + MT100T | - 54 % | nd | 11,65 | nd |
| 3 Eutanol G + MT100T | - 63 % | nd | 8,99 | nd |
| 4 Jojoba + MT100T * | + 46 % | + 99 % | 8,51 | 19,1 |
| 5 Witconol APM + MT100T | - 62 % | nd | 5,74 | nd |

* huile polaire - comparatif

FINSOLV est un benzoate d'alcools en $C_{12}$-$C_{15}$ vendu par la Société FINETEX.

EUTANOL G est de l'octyldodécanol vendu par la Société HENKEL.

WITCONOL APM est de l'alcool myristique oxypropyléné à 3 moles d'oxyde de propylène vendu par la Société WITCO.

<u>EXEMPLE 3</u>

On prépare la composition suivante :

- Alcool cétostéarylique et cétostéareth        5%

- Glycéryl distéarate et glycéryl stéarate        1%

- Alcool cétylique        1%

- Huile de jojoba        6%

- Vitamine F        6%

- Pigment (différents pigments ont été utilisés, conforme tableau II) 3a à 3c        5%

- Eau        qsp        100

Cette préparation est faite selon le schéma décrit à l'exemple 1.

TABLEAU II

EVOLUTION DE LA VITAMINE F EN PRESENCE DE DIVERS PIGMENTS

| Compositions testées | | Pourcentage d'activation (+) ou d'inhibition (-) de l'oxydation de la vitamine F après différents traitements (Exprimé par rapport à la base sans pigments) | | AE BLEUISSEMENT | |
|---|---|---|---|---|---|
| | | 1 h UVA | 24 h UVA | Après 1 h UVA | Après 6 h UVA |
| 3a | MT 100T | + 1738 % | - 44 % | 10,31 | 12,46 |
| 3b | M 262 | + 731 % | - 23 % | 3 | 8 |
| 3c * | P 25 | + 5652 % | + 119 % | 21,24 | 21,65 |

* non conforme à l'invention.

Le **TABLEAU II** montre l'évolution de la composition conforme à l'exemple 3 en utilisant différents pigments.

On constate que le nanopigment P25, qui est un nanodioxyde de titane ayant une granulométrie de 20 nm, commercialisé par la Société DEGUSSA et qui ne porte pas de revêtement d'aluminium et de silicium, est très réactif. L'utilisation de nanopigment revêtu tel que le produit MT100T qui est un nanooxyde de titane (Rutile traité avec du stéarate d'aluminium 80/20) ayant une granulométrie de 15 nm, vendu par la Société TAYCA ou le produit dénommé UV-Titan M262 qui est un nanooxyde de titane (Anatase traité par l'alumine, silice 6,3/1) de granulométrie 20 nm enrobé de 2% de PDMS, commercialisé par la Société KEMIRA.

EXEMPLE 4

Ces compositions sont préparées à partir de la composition de l'exemple 3 contenant comme pigment le produit MT 100T, avec introduction d'un anti-oxydant.

TABLEAU III

| Exemple 3 au MT 100T | QSP - 100 | QSP - 100 |
|---|---|---|
| Acide déhydroascorbique | 1% | - |

Suite du Tableau sur la page suivante

TABLEAU III   (suite)

| | | |
|---|---|---|
| Etoxyquine | - | 1% |
| Bleuissement après 1 heure UVA | 3,35 | 1,95 |
| % d'activation ou inhibition de l'oxydation de la vitamine F en % par rapport à l'exemple 3 au MT 100T :<br>    - 1 h UVA<br>    - 1 h UVA + 5 jours à 37°C | <br><br>- 100%<br>- 93% | <br><br>- 100%<br>- 100% |

EXEMPLE 5

L'exemple suivant est destiné à illustrer la mise en oeuvre de l'invention avec des mélanges d'huiles plus ou moins polaires.

Cet exemple est basé sur la composition de l'exemple 2.

Le **TABLEAU IV** illustre les résultats obtenus.

TABLEAU IV

| | | |
|---|---|---|
| Mélange de deux huiles de polarités différentes dans l'émulsion à la vitamine F contenant 5% de MT 100T | | |
| Huiles en mélange Huile de vaseline (peu polaire)<br>(% pds dans la formule) Ester benzylique (polaire) | 4,5 %<br>1,5 % | 1,5 %<br>4,5 % |
| Bleuissement (ΔE) après 1 heure d'exposition au SUNTEST | 3 | 4 |

**Revendications**

1. Composition cosmétique, caractérisée par le fait qu'elle contient des nanopigments de dioxyde de titane enrobés avec au moins un dérivé d'aluminium dans un milieu cosmétiquement acceptable, peu polaire, ayant une conductivité inférieure à 100 μSiemens et comportant au moins une phase grasse.

2. Composition selon la revendication 1, caractérisée par le fait que les nanopigments de dioxyde de titane sont enrobés avec des dérivés d'aluminium et de silicium.

3. Composition selon la revendication 2, caractérisée par le fait que le nanopigment est enrobé par un mélange d'oxyde d'aluminium et d'oxyde de silicium, l'aluminium et le silicium constituant au moins 5% en poids par rapport au poids de dioxyde de titane et la quantité d'aluminium est supérieure à la quantité de silicium.

4. Composition selon l'une quelconque des revendications 1 à 3, caractérisée par le fait que le taux d'enrobage du nanopigment est compris entre 5 et 30% et de préférence entre 5 et 20% en poids par rapport au poids du dioxyde de titane.

5. Composition selon l'une quelconque des revendications 2 à 4, caractérisée par le fait que le rapport $Al_2O_3/SiO_2$ est supérieur ou égal à 3 et inférieur ou égal à 8, $Al_2O_3$ étant présent dans une quantité supérieure à la quantité de $SiO_2$ qui est présent dans une quantité égale ou supérieure à 1% en poids par rapport au poids du dioxyde de titane.

6. Composition selon l'une quelconque des revendications 1 à 5, caractérisée par le fait que le dioxyde de titane comporte un enrobage supplémentaire facilitant la dispersion du pigment dans le milieu.

7. Composition selon la revendication 6, caractérisée par le fait que l'enrobage supplémentaire est constitué par des silicones, des acides aminés, des sels d'acides gras autres que les sels d'aluminium, de lécithine.

8. Composition selon l'une quelconque des revendications 1 à 7, caractérisée par le fait que le milieu cosmétiquement acceptable comportant au moins une phase grasse est anhydre ou se présente sous forme d'une émulsion eau-dans-huile ou huile-dans-eau.

9. Composition selon l'une quelconque des revendications 1 à 8, caractérisée par le fait que la phase grasse est constituée par une huile ou un mélange d'huiles ou de corps gras pas ou peu polaire.

10. Composition selon la revendication 9, caractérisée par le fait que la constante diélectrique de l'huile ou du mélange d'huiles ou de corps gras est inférieure à 3.

11. Composition selon la revendication 9, caractérisée par le fait que l'indice de polarité de l'huile ou du mélange d'huiles ou de corps gras est supérieur à 24 mN/m.

12. Composition selon l'une quelconque des revendications 1 à 11, caractérisée par le fait que le corps gras est une huile choisie parmi les hydrocarbures ou les esters et triglycérides courts, ou un mélange de ces huiles.

13. Composition selon l'une quelconque des revendications 1 à 12, caractérisée par le fait que les nanopigments enrobés sont présents dans des proportions comprises entre 0,5 et 30% en poids par rapport au poids total de la composition.

14. Composition selon l'une quelconque des revendications 1 à 13, caractérisée par le fait que les nanopigments de dioxyde de titane enrobés ont une granulométrie inférieure ou égal à 200 nm et de préférence inférieure ou égale à 100 nm.

15. Composition selon l'une quelconque des revendications 1 à 14, caractérisée par le fait que la phase grasse peu polaire constitue 5 à 99,5% en poids par rapport au poids total de la composition.

16. Composition selon l'une quelconque des revendications 1 à 15, caractérisée par le fait qu'elle contient des sels dans des proportions telles que la conductivité du milieu reste inférieure à 100 µSiemens.

17. Composition selon l'une quelconque des revendications 1 à 16, caractérisée par le fait que la composition contient des agents antioxydants.

18. Composition selon l'une quelconque des revendications 1 à 17, caractérisée par le fait que les agents antioxydants sont choisis parmi l'extrait d'huile de sésame, la superoxyde dismutase, la caféine, le cytochrome C, le tertiobutyl hydroxytoluène, l'acide diéthylène triamine pentaacétique, l'éthoxyquine, la lactoperoxydase, l'acide déhydroxy ascorbique.

19. Composition selon l'une quelconque des revendications 1 à 18, caractérisée par le fait que l'agent antioxydant est l'éthoxyquine.

20. Composition selon l'une quelconque des revendications 17 à 19, caractérisée par le fait que l'agent antioxydant est utilisé dans des proportions comprises entre 0,01 et 2% en poids, et de préférence entre 0,01 et 0,05% en poids.

21. Procédé de préparation d'une composition telle que définie dans l'une quelconque des revendications 1 à 20, caractérisé par le fait que l'on introduit les nanopigments enrobés dans la phase grasse de la composition, cette phase grasse étant ensuite introduite si nécessaire dans la phase aqueuse.

22. Procédé de prévention ou d'atténuation de la photoréactivité de nanopigments de dioxyde de titane dans des compositions cosmétiques, caractérisé par le fait que l'on procède au revêtement des nanopigments de dioxyde de titane avec au moins un dérivé d'aluminium et qu'on introduit le dioxyde de titane ainsi revêtu dans un milieu cosmétiquement acceptable, peu polaire, ayant une conductivité inférieure à 100 µSiemens.

23. Procédé selon la revendication 22, caractérisé par le fait que les nanopigments enrobés et que le milieu cosmétiquement acceptable sont tels que définis dans l'une quelconque des revendications 1 à 20.

24. Procédé de traitement cosmétique de la peau, caractérisé par le fait que l'on applique sur la peau au moins une composition telle que définie dans l'une quelconque des revendications 1 à 20.

**Office européen des brevets**

# RAPPORT DE RECHERCHE EUROPEENNE

Numero de la demande

EP 95 40 2034

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | GB-A-2 205 088 (TIOXIDE GROUP PLC) *Document* | 1-24 | A61K7/00 |
| A | EP-A-0 518 773 (L'OREAL) * page 13 - page 14; revendications 1-11 * | 1 | |
| A | EP-A-0 518 772 (L'OREAL) * page 15 - page 16; revendications 1-9 * | 1 | |
| A | WO-A-92 21315 (L'OREAL) * page 12; revendications 1-6 * | 1 | |
| A | DE-A-38 24 999 (THE BOOTS CO. PLC) *Document,particulièrement revendication 4* | 1 | |
| D,A | & GB-A-2 217 987 (THE BOOTS CO. PLC) | | |
| D,A | JP-A-3 115 211 (KAO CORP) *Document* | 1-24 | |

**DOMAINES TECHNIQUES RECHERCHES (Int.Cl.6)**

A61K

Le présent rapport a été établi pour toutes les revendications

| Lieu de la recherche | Date d'achèvement de la recherche | Examinateur |
|---|---|---|
| LA HAYE | 2 Novembre 1995 | Luyten, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................
& : membre de la même famille, document correspondant

EPO FORM 1503 03.82 (P04C02)